# EUROPEAN PATENT APPLICATION

(11) **EP 1 985 585 A1**
(43) Date of publication of application: **29.10.2008**
(21) Application number: 06841737.7
(22) Date of filing: 13.12.2006
(51) Int. Cl.: C01B 33/44, C08K 3/34, C08K 7/26, C08K 9/04

(54) **METHOD FOR PRODUCING NANOCOMPOSITE MATERIALS FOR MULTI-SECTORAL APPLICATIONS**

(30) Priority: 29.12.2005 ES 200503232
(71) Applicant: Nanobiomatters, S.L., 46980 Paterna (Valencia) (ES)
(72) Inventor: LAGARON CABELLO, José Maria, E-46980-PATERNA-Valencia (ES); GIMENEZ TORRES, Enrique, E-46980-PATERNA-Valencia (ES); CABEDO MAS, Luis, 46980-PATERNA-Valencia (ES)
(74) Representative: Carvajal y Urquijo, Isabel
(86) International application number: PCT/ES2006/000685
(87) International publication number: WO 2007/074184

(57) **Abstract**

The present invention relates to a process for manufacturing intercalated nanocomposites based on organic compounds with layered structures, **characterized in that** it comprises the following steps:
- reducing the size of the layered particles and purifying them;
- pre-treating the natural and/or synthetic layered structures by means of using precursors;
- intercalating modifiers in the layered structure;
- adding the product resulting from the previous step in liquid form during the processing of a plastic matrix and/or precipitating the product resulting from the previous step to obtain a masterbatch; and
- incorporating the masterbatch into a plastic matrix by any plastics processing method.

## Description

The present invention relates to a process of manufacturing nanocomposite materials with improved gas and vapor barrier properties and thermal and mechanical properties, with antimicrobial and active and bioactive compound release properties and which is biodegradable. The gas barrier properties of plastic materials, preferably polyester and polar polymers, derived both from petroleum and from biodegradable materials from renewable and nonrenewable sources, are substantially improved by means of the synthesis process for the proposed nanocomposite materials.

### Background of the Invention

A huge effort has been made in recent years in nanomaterial, and particularly in nanocomposite, research. Nanocomposites are polymers reinforced with nanoscopic sized filler (i.e. having dimensions which are in at least one direction of the order of one nanometer up to tens of nanometers). Dispersion by means of exfoliation and intercalation of this type of inorganic particles in a polymer matrix allows obtaining a series of new properties that are not shared by conventional materials, such as microcomposites.

Nanocomposites are formed by separating layers by means of different processes giving rise to intercalated or exfoliated structures. The terms exfoliation and intercalation of nanocomposites are described in patents US6384121B1, WO0069957, US5844032, US6228903B1, US2005/0027040A1, W09304118A1. In these structures, the polymer chains are inserted between nanofiller layers or they even completely disperse the initial clay layers between the polymer chains, increasing the mechanical and barrier features.

There have been advances in the literature in relation to nanocomposites made of hybrid organic-inorganic materials which are focused on the synthesis of these materials or on a specific application thereof (earlier references). This great interest is the result of the unique properties of these composites which are often related to the essential role of interfacial forces and surface chemistry as the size of the dispersed phase decreases until reaching nanometric scales. The mechanical, adhesive, cohesive, electrical, optical, photochemical, catalytic and magnetic properties of these hybrid materials frequently result from the synergistic combination of the properties that the constituents alone have. An organic polymer can be made to have greater tensile strength, elasticity, low surface energy, greater hardness, chemical resistance, resistance to radiation and to heat, as well as the inclusion of functional or catalytic groups, by means of interpenetration, inclusion or dispersion of an inorganic component into said organic polymer. Hydrophilic-hydrophobic, covalent or coordination interactions in this type of materials allow stabilization of incompatible phases with a high interface area. It is important to differentiate at this point between guest-host systems, such as intercalated structures for example, in which each component alters the structure of the other, or authentic nanocomposites in which the size of the dispersed phase or nanofiller is such that each component retains its specific structure and properties, although with important properties derived from the small size and large interface, could also be mentioned.

The definition of a hybrid material is therefore rather broad and ranges from single-phase polymeric networks in which the hybrid composition refers to the presence of functional groups or substituents of a different kind in relation to the main component, to guest-host or self-assembled superstructures.

Despite the fact that there are earlier inventions that make use of specific modifications to clays to generate nanocomposites as in patent US6384121B1, even by means of melt mixing routes, these inventions propose modifiers essentially based on quaternary ammonium salts which can lead to different hydrocarbons which in many cases are substances that are forbidden from coming into contact with food and which further do not lead to good compatibility with many families of polymers, or they react during processing. The improvements herein proposed are not described (in reference EP 0 780 340 B1) in the examples in which nanocomposites have been proposed to increase the barrier properties. It is also generally found that most nanocomposites are developed to increase matrix rigidity; however, in many biodegradable materials it is more important to plasticize the material given that they are generally excessively rigid materials which need plasticizers in many applications. There is also great interest in providing nanomaterials which can be of use in biomedical and pharmaceutical applications because they are biocompatible and biodegradable, which improve the properties of the matrix, and being able to design them to control the release of active substances in, for example, active packaging applications and bioactive applications, which release functional substances into foods, and in biomedical and pharmaceutical applications. For this reason there is a need to find improved processes for manufacturing nanocomposites which reduce costs, production times thereof, which improve the properties without jeopardizing the quality of the end product and which allow their use for different matrixes and applications to be optimized.

### Description of the Invention

The present invention provides a new route for manufacturing nanocomposites which gives rise to an end product with improved gas and vapor barrier properties, which is biodegradable and has either antimicrobial properties or with the capacity for the controlled release of active or bioactive substances such as antimicrobial agents, antioxidants, ethylene, ethanol, drugs, bioavailable calcium compounds and mixtures thereof. It also enables rigidizing or plasticizing the matrix depending on the formulation and it further makes use of substances which can come into contact with food and/or substances approved for biomedical and pharmaceutical use, thus improving the quality of the end product and offering new properties and improvements in relation to the prior state of knowledge and solving the problems described in the state of the art.

The novel process for manufacturing the nanocomposites described in the present invention, which can be based on structures such as layer phyllosilicates, including clays (e.g. montmorillonite, kaolinite, bentonite, smectite, hectorite, sepiolite, saponite, halloysite, vermiculite, mica) or synthetic or natural layered double hydroxides with a layered structure which are intercalated with organic type with materials, comprising the following steps:
Reducing the size of the layered particles by means of mechanical action and the subsequent vibrating screen filtering process until reaching an interval comprised from 0.1 to 100 microns, and according to a preferred embodiment of the present invention the reduction obtains a particle size of less than 25 microns. After the filtering process, the organic matter is removed by decanting, collection of supernatant or by chemical reaction with oxidizing substances such as peroxides, and the crystalline oxides and hard particles not subject to modification are finally removed either by means of centrifugation processes and/or gravimetric processes in solution or turbo-drying processes, preferably by an atomization process with controlled pressure reduction. The thin layers thus obtained are considered to be the starting materials of the present invention.

A next step in the process is the pre-treatment of the layered structures in one or in several passes by means of using the expansive-type precursors shown in Table 1, and preferably DMSO, alcohols, acetates, or water and a mixture thereof, which activate the fining agents by means of an initial increase of the basal spacing of the layers and modify the surface features of the clay. The penetration of the precursors is accelerated by means of the use of temperature, a turbulent regime homogenizer, ultrasound, pressure or a combination thereof. They can be dried by evaporation in a stove, lyophilization, centrifugation processes and/or gravimetric processes in solution or turbo-drying processes or by atomization processes. According to another preferred embodiment of the present invention, the solution of the intercalated precursor can be used without a prior drying process as a starting means from the following step of incorporating the modifier.

Another subsequent step in the process for manufacturing intercalated nanocomposites based on organic compounds with layered structures consists of intercalating the organic materials in the layered structure in an aqueous base or with polar solvents. According to a preferred embodiment of the present invention, the organic compounds can be PVOH, EVOH and derivatives of the same family, and/or biopolymers such as peptides and natural or synthetic proteins obtained chemically or by genetic modification of microorganisms or plants and natural or synthetic polysaccharides obtained chemically or by genetic modification of microorganisms or plants and polypeptides, nucleic acids and synthetic nucleic acid polymers obtained chemically or by genetic modification of microorganisms or plants, and biodegradable polyesters such as polylactic acid, polylactic-glycolic acid, adipic acid and derivatives thereof, and polyhydroxyalkanoates, preferably polyhydroxybutyrate and their copolymers with valerates and biomedical materials, such as hydroxyapatites. When the organic material that is intercalated is EVOH or any material of the family thereof with molar contents of ethylene preferably less than 48%, and more preferably less than 29%, they can be taken to saturation in aqueous medium or in specific alcohol-type solvents and mixtures of alcohols and water, more preferably of water and isopropanol in proportions by volume of water greater than 50%. According to another preferred embodiment of the present invention, biopolymers with or without plasticizers, with or without crosslinkers and with or without emulsifiers or surfactants or another type of additives, are from the group of synthetic and natural (plant or animal) polysaccharides such as cellulose and derivatives, carrageenans and derivatives, alginates, dextran, gum arabic and preferably chitosan or any of both its natural and synthetic derivatives, more preferably chitosan salts and even more preferably chitosan acetate, and both proteins derived from plants and animals and proteins from maize (zein), the gluten derivatives, such as gluten or its gliadin and glutenin fractions, and more preferably gelatin, casein and soy proteins and derivatives thereof, as well as natural or synthetic polypeptides preferably of the elastin type obtained chemically or by genetic modification of microorganisms or plants, hexadecyltrimethylammonium bromide and mixtures thereof. In the case of chitosan, the deacetylation degree will preferably be higher than 80% and more preferably higher than 87%. The penetration of the precursors will be accelerated by means of the use of temperature, a turbulent regime homogenizer, ultrasound, pressure or a combination thereof.

Low molecular weight substances having an active or bioactive nature will be added in a step that is subsequent or alternative to dissolving the fining agents pretreated with the previously proposed precursors and modifying agents for the purpose of either being intercalated or released in a controlled manner, giving rise to nanocomposites with an active or bioactive capacity. The active substances will be ethanol, or ethylene, or of the essential oil type, preferably thymol, carvacrol, linalol and mixtures, or natural small-sized antimicrobial peptides (bacteriocins) or those obtained by genetic modification, preferably nisins, enterocins, lacticins and lysozyme or natural or synthetic antioxidants, preferably polyphenols, preferably flavonoids, rosemary extract and vitamins, preferably ascorbic acid or vitamin C, or drugs, or bioavailable calcium compounds bioavailable calcium compounds. It is expected that these elements can be released from the nanocomposite towards the product in a controlled manner (matrix control) and exert their active or bioactive role, that they can be released from the matrix and that the nanoparticles will control the kinetics (nanoadditive control) or it can be released from both. The contents to be added are generally less than 80% by volume of the solution, preferably less than 12% and more preferably less than 8%. The penetration of these substances is accelerated by means of the use of temperature, a turbulent regime homogenizer, ultrasound, pressure or a combination thereof.

Another step of the present invention is to add the product resulting from the previous steps in liquid state to a plastic matrix. In this case it is added to the plastic matrix during the processing thereof using any method of manufacture related to the plastic processing industry, such as extrusion, injection, blowing, compression molding, resin transfer molding, calendering, thermal shock, internal ultrasonic mixing, coextrusion, co-injection and a combination thereof. According to a preferred embodiment, the plastic matrix is preferably made of PVOH, EVOH or derivatives and biodegradable materials such as proteins, polysaccharides and polyesters, and biomedical materials such as hydroxyapatites or mixtures of all the foregoing and can contain any type of additives typically added to plastics to improve their processing or their properties.

According to another preferred embodiment of the present invention, the product resulting from the previous steps is precipitated by evaporation using drying methodologies such as heating and/or centrifugation processes and/or gravimetric processes in solution or turbo-drying processes and/or atomization processes; by cooling or by adding a precipitating agent to form a masterbatch, i.e. an additive concentrate, which is triturated to give rise to a particulate product by grinding and/or it is processed by means of any plastics processing method to obtain pellets in solid state. In this same sense, the masterbatch is directly used to obtain an end product by means of any process of manufacture related to the plastics processing industry, such as extrusion, injection, blowing, compression molding, resin transfer molding, calendering, thermal shock, internal ultrasonic mixing, coextrusion, co-injection and a combination thereof, or it is used as a diluted additive in the same or in another plastic matrix (including the aforementioned biopolymers and biomedical materials) in a conventional plastics processing route such as those mentioned above.

According to the process described in the present invention, the intercalated nanocomposites based on organic materials with layered structures are applied for reinforcing plastics in packaging applications in general and in food and food component packaging applications in particular, for biomedical applications as nanobiocomposites and in pharmaceutical applications for releasing active ingredients, as a barrier to solvents and organic products, such as aromas and aroma components, oils and hydrocarbons, and to mixed organic and inorganic products, for applications requiring a biodegradable or compostable character, for active packages requiring antimicrobial, antioxidant character, or of another type, requiring the controlled release of low molecular weight, preferably volatile, substances, for applications requiring antimicrobial capacity and for the use of biopolymers either without needing to use plasticizers or needing lower amounts of the latter.

All the features and advantages set forth as well as other features and advantages of the invention can be better understood with the following examples. Furthermore the examples are illustrative rather than limiting so as to be able to better understand the present invention.

### Examples

### EXAMPLE 1: EVOH ROUTE

In this example the modification process consists of a first step in which the purified kaolinite and montmorillonite clay fining agents are pretreated with an ethanol/water 50/50 (v/v) mixture at 50°C. This process was carried out together with an ultrasonic stirring treatment process for 1 hour and with stirring with a homogenizer for 2 hours to favor the intercalation of the precursor in the clay. The solvent was then removed by means of lyophilization and/or evaporation. In another example an aqueous solution of DMSO was used as a precursor, an even greater expansion of the clay being obtained as is described in Table 1.

**Table 1**

| **MODIFIER** | **d_{MODIFIER} (nm)** | **MODIFIER** | **d_{MODIFIER} (nm)** |
|---|---|---|---|
| *Unmodified kaolinite* | 0.72 | *Unmodified montmorillonite* | 0.98 |
| *Dimethyl sulfoxide (DMSO)* | 1.11 | *Polyethylene oxide* | 1.12 |
| *N-methyl formamide (NMF)* | 1.02 | *Cellulose acetobutyrate* | 1.13 |
| *Hydrated hydrazine* | 1.03 | *Calcium butyrate* | 0.92 |
| *Water* | 0.78 | *Sucrose acetoisobutyrate* | 1.08 |
| *Alcohols* | 1.10 | *Manganese butyrate* | 0.95 |
| *Anhydrous hydrazine* | 0.96 | *Carboxymethyl starch* | >3 |
| *Acetamide* | 1.09 | *Starch* | 1.21 |
| *DMSO+Methanol (MeOH)* | 1.12 | *Hydroxyethyl starch* | 1.15 |
| *Hexanoic acid* | 1.23 | *Hydroxypropyl starch* | 1.14 |
| *Acrylamides* | 1.44 | *Adonitol* | 1.04 |
| *Glucose* | 1.25 | *Sorbitol* | 1.19 |
| *Archylamide* | 1.14 | *Dibenzylidene sorbitol* | 1.16 |
| *Salicylic acid* | 1.07 | *Ethylene glycol* | 0.95 |
| *Manganese acetate* | 1.41 | *Polypropylene glycol* | 1.01 |
| *Caprolactam* | 1.18 | *Propylene glycol* | 1.01 |
| *Vinyl acetate* | 1.21 | *Glycolic acid* | 1.06 |
| *Potassium acetate* | 1.39 | *Triethylene glycol* | 1.08 |
| *Tannic acid* | 1.09 | *Tetraethylene glycol* | 1.06 |
| *Maleic acid* | 1.20 | *Glycerol* | 1.02 |
| *Maleic anhydride* | 1.20 | *1,2-Propanediol* | 1.09 |
| *Lactic acid* | 1.08 | *1,3-Propanediol* | 0.98 |
| *Adipic acid* | 1.03 | *Polyethylene glycol M_{w}=1000* | 1.11 |
| *Acetic acid* | 1.10 | *Polyethylene glycol M_{w}=3400* | 1.12 |
| *Acetaldehyde* | 0.91 | *Sorbitan* | 1.09 |
| *Butyric acid* | 1.01 | *Dipropylene glycol* | 1.03 |
| *Tetrafluoroethylene* | 0.98 | *Diethylene glycol* | 1.04 |
| *Chlorotrifluoroethylene* | 1.05 | *Vinyl pyrrolidone* | 1.23 |
| *Hexamethylene* | 1.02 | *Vinyl versatate* | 1.11 |

In addition, an isopropanol/water 70/30 (v/v) solution with EVOH26 (26% molar ethylene content) was prepared and in another example a PVOH aqueous solution was prepared. In both cases, the suspension process was carried out together with an ultrasonic stirring treatment for 1 hour and with stirring by means of a homogenizer for 1 hour.

The pretreated clay dust was then added until reaching saturation conditions, approximately 40% by weight of clay, in one case to the isopropanol/water 70/30 (v/v) solution with EVOH26 (26% molar ethylene content) and in another example to the PVOH aqueous solution. The precipitated product obtained when saturation conditions are reached either by removing the solvent or by cooling is referred to as masterbatch or concentrate. In another example, the previous solutions or solutions that are more diluted in terms of their clay content were, instead of precipitated, added in liquid state to a mold from which nanocomposite films were obtained by evaporating the solvent.

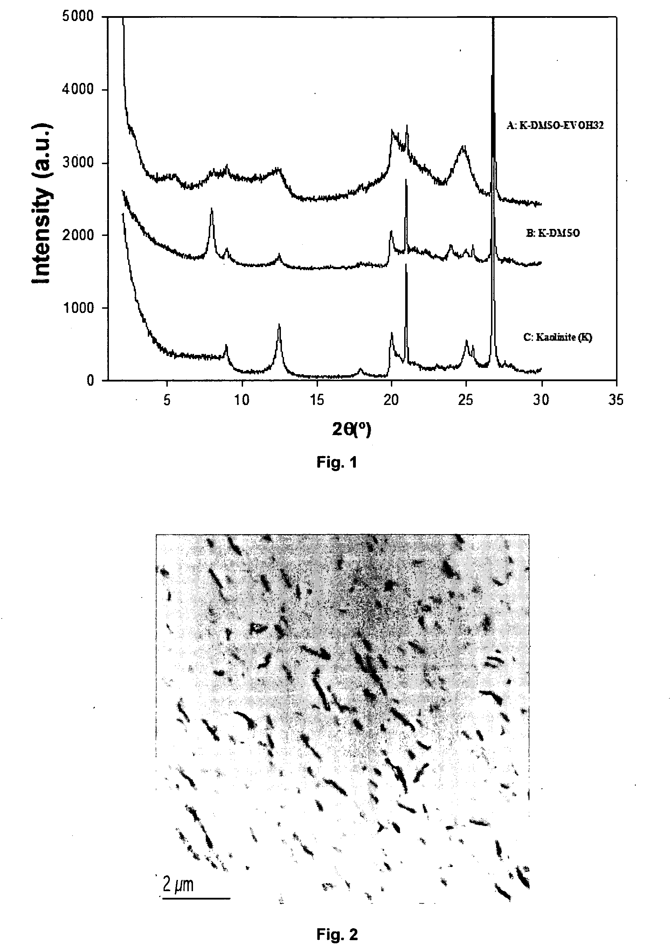

In the sample treated with DMSO, see the diffractogram in Figure 1, it can be seen that the DMSO precursor agent leads to an expansion of the interlayer spacing of the clay towards lower angles; this indicates an expansion of the clay layers by intercalation. In the masterbatch finally obtained with this clay modified as described in the preceding protocol, a high degree of intercalation with the polymer can be seen as it is derived from the observation of different peaks at lower angles. The masterbatch thus obtained was melt extruded or mixed in an internal mixer at a temperature of 210°C and 100 rpm for 5 minutes maximum with pure EVOH32 to obtain a nanocomposite with 4% by weight clay content.

In an alternative process to the preceding process, a more diluted version of the masterbatch solution (<10% weight in day) was added to a melt extrusion or mixture process with pure EVOH32 to obtain a 4% by weight EVOH/clay nanocomposite, using for that purpose an internal mixer at a temperature of 210°C and 100 rpm for 5 minutes maximum. In another example, the clay solution treated with the precursor was directly added to a melt extrusion or mixing process. The most satisfactory case out of all of them was the case of the masterbatch either precipitated or added in liquid state to a melt extrusion or mixing process.

75 micron-thick sheets were prepared using the extracted material of the melt processing using a hot platen press. The molding conditions used were temperature of 220°C and pressure of 2 MPa for 4 minutes. The sheets obtained were used for the morphological and mechanical characterization and for the characterization of the barrier properties. Figure 2 shows a TEM microphotograph indicating that a morphology is obtained with a high degree of exfoliation/intercalation of this nanocomposite, where the disperse clay nanoflakes can be seen with a darker color in the polymer matrix.

The carrier properties measured in films of the samples obtained from the masterbatch diluted by hot mixing are shown in Table 2.

**Table 2**

| ***Material*** | ***Oxygen permeability m³ m*/*m² s Pa*** |
|---|---|
| Amorphous PLA, 24°C, 0% RH | 1.22x10⁻¹⁸ |
| NanoPLA 4% Kaolinite, 24°C, 0% RH | 6.40x10⁻¹⁹ |
| (Nanoter® 02 of Nanobiomatters S.L. modified with chitosan) | 48% reduction |
| NanoPLA 4% Montmorillonite, 24°C, 0% RH | 9.89x10⁻¹⁹ |
| (Southern Clay Inc. Cloisite® 20A is a montmorillonite modified with quaternary ammonium salts) | 19% reduction |
| EVOH 45^{°C}, 0% RH | 4.00x10⁻²¹ |
| EVOH 4% Kaolinite, 45°C, 0% RH | *Under 1.00x10⁻²¹ |
| (Nanoter® 01 of Nanobiomatters S.L., masterbatch route) | At least 75% reduction |
| EVOH 24°C, 85% RH | 7.00x10⁻²¹ |
| EVOH 4% Kaolinite, 24°C, 85% RH | 2.00x10⁻²¹ |
| (Nanoter® 01 of Nanobiomatters S.L., masterbatch route) | 71% reduction |
| EVOH 24°C, 94% RH | 9.00x10⁻²¹ |
| EVOH 4% Kaolinite, 24°C, 94% RH | 3.00x10⁻²¹ |
| (Nanoter® 01 of Nanobiomatters S.L., masterbatch route) | 66.7% reduction |
| PCL, 24°C, 0% RH | 5.50x10⁻¹⁸ |
| PCL 4% Montmorillonite, 24°C, 0% RH | 4.50x10⁻¹⁸ |
| (Nanoter® 03 of Nanobiomatters S.L., modified with soy protein) | 18.2% reduction |
| PHB, 24°C, 0% RH | 4.12x10⁻¹⁹ |
| PHB 4% Montmorillonite, 24°C, 0% RH | 2.10x10⁻¹⁹ |
| (Nanoter® 02 of Nanobiomatters S.L. modified with chitosan) | 49.1% reduction |
| PHB 4% Montmorillonite, 24°C, 0% RH | Inconsistent sample, the route is |
| (Southern Clay Inc. Cloisite® 20A is a montmorillonite modified with quaternary ammonium salts) | unsuitable for this modification |
| HDPE, 24°C, 0% RH | 5.10x10⁻¹² |
| HDPE, 7% Kaolinite, 24°C, 0% RH | 4.05x10⁻¹² |
| (Nanoter^{®} of Nanobiomatters S.L. modified with hexadecyltrimethylammonium bromide) | 20.6% reduction |

| | |
|---|---|
| *Measurement under the limit of detection of the permeability equipment | |

Table 2 shows that the barrier properties against oxygen are much better in the nanocomposites and have a much higher value than what was reported in the earlier patent or scientific literature on nanocomposites.

### EXAMPLE 2: BIOPOLYMER ROUTE

The modification process followed in this example consists of a first modification step of the kaolinite and montmorillonite clay fining agents by means of an aqueous solution treatment of potassium acetate and DMSO as precursors. This process was carried out together with an ultrasonic stirring treatment process for 1 hour at 50°C and with stirring by means of a homogenizer for 2 hours to favor the intercalation of the precursor in the clay.

In addition, in one example a chitosan aqueous solution was suspended at 40°C (100 ml of water and 2 ml of acetic acid were required for every 1 g of chitosan), and in another example soy protein aqueous solution was suspended at 45°C. In both cases, the suspension process was carried out together with an ultrasonic stirring treatment for 1 hour and with stirring by means of a homogenizer for 1 hour.

The suspension of the clay modified with the precursor was then added in one example to the chitosan aqueous solution at a (2:1) biopolymer clay by weight ratio and in another example it was added to a soy protein aqueous solution in at a (2:1) biopolymer clay by weight ratio. This process was carried out together with ultrasonic stirring treatment for 1 hour and with stirring by means of a homogenizer for 1 hour to favor the intercalation of the biopolymer in the clay. The solvent was then removed by a lyophilization and/or evaporation process. The nanocomposites were finally obtained using a single screw extruder or internal mixer at a temperature of 110°C for PCL (polycaprolactone of Solvay, Belgium) and 190°Cfor PLA (amorphous polylactic acid of Galactic, Belgium) and PHB (plasticized polyhydroxybutyrate of Goodfellow, Great Britain) and of 100 rpm for 5 minutes maximum.

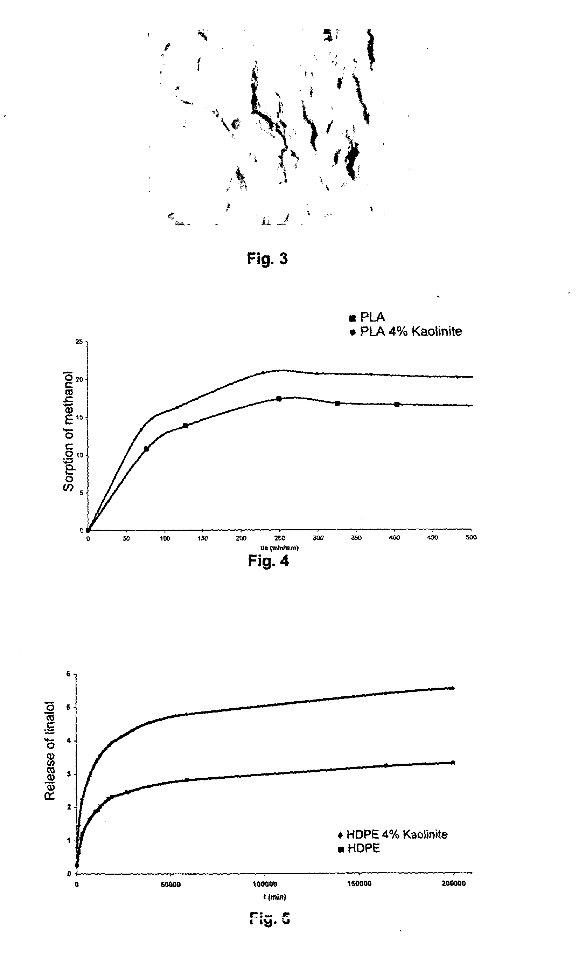

In another example the solution of the clay treated with the precursors is added to a solution of the biopolymers, is subjected to homogenization and/or ultrasound for one hour, is then added to a mold and by evaporating the solvent a nanocomposite film is obtained (see Figure 3). Figure 3 shows a morphology with a high degree of exfoliation/intercalation of a PCL nanocomposite obtained by evaporation from a chloroform solution, where the disperse clay nanoflakes can be seen in a darker color in the matrix.

700-micron thick sheets were prepared using the extracted material of the melt process using a hot platen press. The sheets obtained were used for the morphological and mechanical characterization and for the characterization of the barrier properties.

Figure 4 shows the gravimetric sorption of methanol according to the corrected time for the PLA thickness and PLA nanocomposite. The sorption of methanol is used to simulate the retaining capacity of a polar compound with antimicrobial properties in materials. In this figure it can be seen how the nanocomposite retains a greater component amount; this behavior is advantageous because it allows the active and bioactive substance release to be modified and controlled in different applications.

**Table 3**

| ***SAMPLES*** | | ***Modulus of Rigidity E^{*} (Pa)*** | ***Modulus of Rigidity E^{*} (Pa)*** |
|---|---|---|---|
| | | ***T*= -*18°C*** | ***T*= *20°C*** |
| • Pure: | | | |
| | PLA | 5.6x10⁹ | 5.4x10⁹ |
| | PCL | 6.2x10⁸ | 4.4x10⁸ |
| | PHB | 3.4x10⁹ | 1.9x10⁹ |
| | HDPE | 1.3x10⁹ | 1.1x10⁹ |
| • Nanocomposites: | | | |
| | PLA/chitosan | 4.8x10⁹ | 4.5x10⁹ |
| | PCL/soy | 8.5x10⁸ | 6.6x10⁸ |
| | PHB/chitosan | 2.4x10⁹ | 1.7x10⁹ |
| | HDPE/ammoniumC16 | 1.6x10⁹ | 1.4x10⁹ |

Table 3 indicates that in contrast to what would be expected and to what is observed for polyolefins (see HDPE) and PCL, nanobiocomposites of rigid PLA and PHB materials obtained by melt processing show a reduction in the mechanical modulus of rigidity. The mechanical data shown are measured by means of dynamic-mechanical analysis (DMA) under bending. This is surprising and is closely related to the specific interaction that is established between the biomaterials and the additives proposed in this invention. The observation of plasticization in the nanobiocomposite is positive because pure biomaterials usually have excessive rigidity, and their plasticization as a result of the incorporation of the nanoclays of the present invention makes them very suitable for applications in which excessive brittleness of biopolymers is a problem. Table 2 additionally indicates that all the materials, but especially PHB, show improvement regarding the oxygen barrier. Table 2 also indicates that modifications made with ammonium as those described in earlier patent literature lead to smaller permeability reductions, as in the case of PLA or to inconsistent samples such as for the case of the PHB.

The improvement of PHB is very significant and is greater than all the improvements made in the oxygen barrier properties reported to date in the literature.

Like chitosan, alcohols and essential oils are potent antimicrobial and bioactive agents, and it can be expected that clay modified with chitosan and/or containing active and bioactive substances and their films or mixtures have antimicrobial or bioactive capacity. This can be derived from the numerous examples existing in the scientific literature in which these substances have great capacity as antimicrobial and bioactive agents. Again, since all the components and materials are biodegradable and compostable, given the abundance of scientific literature that proves it is to be expected that the combination thereof to make nanobiocomposites is also biodegradable and compostable.

### EXAMPLE 3: POLYOLEFIN ROUTE

The modification process followed in this example consists of a first modification step of the kaolinite and montmorillonite clay fining agents by means of a treatment with a solution of DMSO as a precursor. This process was carried out together with an ultrasonic stirring treatment process for 1 hour at 50°C and with stirring by means of a homogenizer for 2 hours to favor the intercalation of the precursor in the clay. The solvent was then removed by a lyophilization and/or evaporation process to give rise to a powdered product.

In a second step, the clay is again suspended in a hexadecyltrimethylammonium bromide (C16) aqueous solution in the presence of ultrasonic stirring and a homogenizer at 50°C for 4 hours or in a chitosan solution as described above. The solvent was then removed by a lyophilization and/or evaporation process to give rise to a powdered product.

The modified clay is added in powder form to a melt mixing process with HDPE (BP Chemicals) and a compatibilizing agent, such as maleic anhydride (<5% by weight) to obtain an HDPE/clay nanocomposite with 7% by weight of clay, using for that purpose a single screw extruder or an internal mixer at a temperature of 180°C and 80 rpm for 10 minutes.

700-micron thick sheets were prepared using the material obtained by the melt processing using a hot platen press. The sheets obtained were used for the mechanical characterization and for the characterization of the barrier properties.

It can be derived from Table 3 that for the case of polyethylene and modification conditions of the present invention, the mechanical rigidity of the material increases both at low temperatures and at room temperature, indicating improvement in the mechanical properties. It is additionally derived from Table 2 that the nanocomposite shows a significant increase for polyolefins in the oxygen barrier.

Figure 5 shows the linalol release capacity according to time for samples of pure polyethylene of the same thickness and of a nanocomposite by way of example. Linalol is a relatively polar essential oil having antimicrobial properties and therefore is very interesting for those applications requiring the controlled release of antimicrobial agents or of other active or bioactive substances. It can be derived from this figure that like the nanobiocomposites of Figure 4, the nanocomposites retain and therefore release a greater amount of active and/or bioactive substances.

## Claims

1. A process for manufacturing nanocomposites with a layered structure, **characterized in that** it contains intercalated organic materials and comprises the following steps:
a) reducing the size of the layered particles and purifying them;
b) pre-treating the layered structures by means of using precursors;
c) intercalating organic materials in the layered structure;
d) adding the product resulting from the previous step in liquid form during the processing of a plastic matrix and/or precipitating the product resulting from the previous step to obtain a masterbatch; and
e) incorporating the masterbatch into a plastic matrix by any plastics processing method.

2. A process according to claim 1, **characterized in that** the intercalated nanocomposites based on organic materials with layered structures are from the group of layer phyllosilicates and/or synthetic and/or natural layered double hydroxides.

3. A process according to claim 1, **characterized in that** step a) is carried out by means of mechanical action followed by filtering and removing organic matter.

4. A process according to any of the previous claims, **characterized in that** the product resulting from step a) has a size comprised from 0.1 to 100 microns.

5. A process according to claim 4, **characterized in that** the reduction of the layered particle sizes is done to under 25 microns.

6. A process according to claim 1, **characterized in that** step b) is carried out in one step or in several.

7. A process according to claims 1 and 6, **characterized in that** the pre-treatment is carried out with expanding agents.

8. A process according to claim 1, **characterized in that** in step c) the organic materials to be intercalated are PVOH, EVOH.

9. A process according to claim 1, **characterized in that** in step c) the organic materials to be intercalated are from the group consisting of biopolymers with or without additives.

10. A process according to claim 9, **characterized in that** the intercalation of the biopolymers is carried out in polar solvents.

11. A process according to claims 9-10 **charaterized in that** the biopolymers are from the group of polysaccharides, polyesters, peptides, proteins and/or nucleic acids.

12. A process according to claim 11, **characterized in that** the polyesters are from the group of biodegradable polyesters.

13. A process according to claim 11, **characterized in that** the polysaccharides are from the group of chitosan and/or any of its derivatives.

14. A process according to claim 13, **characterized in that** the deacetylation degree of chitosan is higher than 80%.

15. A process according to claim 13, **characterized in that** the deacetylation degree of chitosan is higher than 87%.

16. A process according to claim 11, **characterized in that** the proteins are derived from plants and/or from animals.

17. A process according to claim 11, **characterized in that** the peptides are from the group consisting of elastin.

18. A process according to claim 1, **characterized in that** in step c) the organic materials to be intercalated are of an active and/or bioactive nature from the group consisting of essential oils and/or antioxidants, and/or ethanol and/or ethylene and/or drugs and/or bioavailable calcium compounds and/or bacteriocins.

19. A process according to claim 18, **characterized in that** the essential oils are from the group consisting of thymol, linalol, carvacrol, mixtures thereof.

20. A process according to claim 18, **characterized in that** the bacteriocins are from the group consisting of nisins, enterocins, lacticins and lysozyme.

21. A process according to claim 18, **characterized in that** the antioxidants are from the group consisting of polyphenols and/or rosemary extract and/or vitamins.

22. A process according to claim 18, **characterized in that** the organic material of an active and/or bioactive nature is added in concentrations of less than 80% by volume.

23. A process according to claim 18, **characterized in that** the organic material of an active and/or bioactive nature is added in concentrations of less than 12% by volume.

24. A process according to claim 18, **characterized in that** the organic material of an active and/or bioactive nature is added in concentrations of less than 8% by volume.

25. A process according to claim 8, **characterized in that** the content of EVOH or any material of the EVOH family is less than 48%.

26. A process according to claim 8, **characterized in that** the content of EVOH or any material of the EVOH family is less than 29%.

27. A process according to claim 1, **characterized in that** the plastic matrix of step d) is any plastic matrix including biodegradable and/or biomedical and/or pharmaceutical matrixes.

28. A process according to claim 27, **characterized in that** the plastic matrix can be processed by means of mixing using any plastics manufacturing process.

29. A process according to claim 1, **characterized in that** the product resulting from step
c) is added to a plastic matrix of the same and/or different material in liquid state to obtain an end product by means of any plastics processing method.

30. A process according to claim 1, **characterized in that** the product resulting from step
c) is precipitated and triturated by grinding and/or is processed by means of any plastics processing method to obtain an end product in solid state referred to as masterbatch.

31. A process according to claim 1, **characterized in that** the product resulting from step
c) is precipitated by evaporation.

32. A process according to claim 1, **characterized in that** the product resulting from step
c) is precipitated by cooling.

33. A process according to claim 1, **characterized in that** the product resulting from step
c) is precipitated by adding a precipitating agent.

34. A process according to claim 1, **characterized in that** the masterbatch is used directly during step d) to obtain the end product by means of any plastics processing method.

35. A process according to claim 1, **characterized in that** the masterbatch is used as an additive for the same matrix and/or for another biopolymer matrix in a conventional plastics processing route.

36. A process according to any of the previous claims, **characterized in that** the intercalated nanocomposites based on organic materials with layered structures, are applied to reinforce plastics in packaging applications in general and in packaging of foods and food components in particular, in applications requiring a barrier to solvents and organic products and to mixed, organic and inorganic products and/or for applications requiring a biodegradable or compostable character and/or for active packages requiring an antimicrobial character and/or for any application requiring antimicrobial capacity and/or for the use of biopolymers without needing to use plasticizers and/or with a lower amount thereof, for active and bioactive packages and in the controlled release of active and/or bioactive substances in general and for the biomedical and pharmaceutical fields in particular.
